Europäisches Patentamt

European Patent Office

Office européen des brevets

(19)

(11) Publication number: **0 299 127**
**A1**

# EUROPEAN PATENT APPLICATION

(21) Application number: **87810403.3**

(22) Date of filing: **16.07.87**

(51) Int. Cl.⁴ **C12N 15/00**

(43) Date of publication of application:
**18.01.89 Bulletin 89/03**

(84) Designated Contracting States:
**AT BE CH DE ES FR GB GR IT LI LU NL SE**

(71) Applicant: **IntraCel Corporation**
**c/o Cottle Catford & Co. 17 High Street**
**Bridgetown(BB)**

(72) Inventor: **Bromley, Peter**
**26, chemin du Pont de Ville**
**CH-1224 Chene-Bougeries(CH)**
Inventor: **Dreano, Michel**
**5, Chemin des Bouvreuils**
**CH-1234 Vessy(CH)**
Inventor: **Voellmy, Richard**
**7240 SW 124 Street**
**Miami Florida 33156(US)**

(74) Representative: **Dousse, Blasco et al**
**7, route de Drize**
**CH-1227 Carouge/Genève(CH)**

(54) **A method for the expression of recombinant genes under stimulation from an inducively expressed activator protein.**

(57) A DNA system for the stimulated expression of genes of interest. Said genes are placed under the control of an activator stimulated promoter and the gene coding for this activator is driven by an inducible promoter. The hybrid DNA constructions are incorporated in suitable host cells which, after multiplication and optional gene amplification, are stressed, whereby the activator produced continues to bring about expression of said genes of interest for a long period of time after the inducible promoter has returned to rest.

EP 0 299 127 A1

# A METHOD FOR THE EXPRESSION OF RECOMBINANT GENES UNDER STIMULATION FROM AN IN-DUCIVELY EXPRESSED ACTIVATOR PROTEIN

## INTRODUCTION

The production of proteins of medical and alternative commercial interest by the use of genetically engineered expression systems has been the subject of great interest and research effort over recent years. Since many of the proteins of interest are naturally secreted proteins, they frequently undergo a variety of post-tranlational modifications such as glycosylation, specific chemical modification and complex folding prior to becoming biologically functional macromolecules.

The two major approaches to the expression of human and other Eucaryotic genes **in vitro** have been used: in the first of these, procaryotic producer cells have been employed, most common bacteria such as **Escherichia coli** and to a lesser extent **bacillus Subtillis**. These cellular systems have proved adequate for a high level production of a number of relatively simple polypeptides but, on the other hand, have shown certain limitations, for example the inability of such producer cells to form S-S bridges, to perform glycosylation, etc.; in addition, high level expression has frequently led to accumulation of product in the form of insoluble particles inside the bacteria. Somewhat complex renaturation steps are required to produce active protein from these insoluble particles and the final product is frequently a mixture of structural forms of the natural macromolecule.

A second approach has been to employ Eukaryotic producer cells for the production of Eukaryotic proteins. The simplest application of such cell types has been the use of yeast where there is the advantage that a great deal of prior fermentation experience already exists. There are a number of examples of successful protein production in yeasts such as a Hepatitis B surface antigen subunit vaccine, interferons, interleukins, etc. Limitations exist both at the level of secretion of protein products and due to post-translational modification reactions that are specific to yeast, and different from that existing in the natural producer cell type for the protein in question.

With these objectives in mind, the use of the expression control elements of heat shock genes to drive the expression of genes was proposed by Bromley and Voellmy for the first time in March 1981 and disclosed in Patent Application US serial No. 628.588. The reasons that led to this development were that heat shock genes themselves are expressed at exceedingly high levels in heat-treated cells, and that these genes are subject to an extremely tight regulation of expression, i.e. they are highly active in stressed cells but essentially inactive under normal conditions of cell growth. Thus, at this time it seemed possible to use the transcription and translation control elements of heat shock genes to drive the expression at high level of synthesis, of genes of interest. This concept was later entirely confirmed and became the object of several pulications and patent applications (see thereafter).

Further developments in the techniques of using heat shock sequences for regulating the expression of foreign genes (e.g. structural genes), i.e. incorporating recombinant DNA constructs into suitable prokaryote and eukaryote host cells and subjecting the cultures to stress have been disclosed in PCT EP86/00451. We now wish to describe a new heat inducible protein expression instrument and method which allow to substancially prolong the time of expression and the efficiency of a gene driven by a foreign regulatory element.

## SUMMARY OF THE INVENTION

In the experiments described hereafter we demonstrate in transient expression assays that the expression of a gene of interest can be regulated by the **trans**-activator tat-III protein of the Human Immunodeficiency virus (HIV) causative agent of the acquired immunodeficiency syndrome, (AIDS), itself synthesized under the transcriptional control of a heat shock promoter. For several reasons to be explained, this type of expression system promises to be superior to the more conventional system in which the expression of a gene of interest is directly regulated by a linked heat shock promoter.

In initial series of experiments disclosed earlier (see the previous references), we linked heat shock regulation elements from a **Drosophila** hsp70 gene as well as from a human hsp70 gene (Voellmy et al., (1985) PNAS 82, 4959-4953) to a variety of test genes including, **E. coli**, -b-galactosidase, human growth hormone (hGH). chicken lysozyme and an Influenza virus haemagglutinin gene, and we have shown that the protein products of these genes are made heat-inducible in a variety of cells (Lawson et al., (1984) Mol.

Gen. Genet. 198, 116-124; Dreano et al., (1987) Gene 49, 1-8). For instance, permanent mouse cell lines carrying stably integrated copies of a heat shock-hGH gene construct were shown to produce hGH in a heat-inducible fashion at levels of expression of the hormone that compare well with those obtained by others using alternative promoter systems (Dreano et al., (1987) Gene 49, 1-8; Dreano et al., (1987) Virus Research, in press).

However, continuous production of selected proteins requires the use of multiple cycles of heat activation; although heat shock genes respond rapidly to a specific stress, such as heat, these genes revert to their normal inactive mode as soon as the stress is removed. Protein product synthesis however does not cease quite as rapidly. Synthesis may indeed continue for a period of 24 hours or so, beyond the period of active transcription (depending on the half life of the mRNA of the gene of interest). For details of repeated heat shocks applied to protein production, see Dreano et al., (1987) Gene 49, 1-8. However, for production purposes, synthesis may need to be maintained over a much longer period (one or more weeks, for example). To achieve this result, cultures of producer cells should be subjected to periodic heat treatments which appear to have the following shortcomings:

a) it is technically inconvenient to periodically raise the fermentator temperature for a great number of cycles, especially with large scale cultures, because present industrial equipment is not adapted accordingly and introducing modified systems would be expensive

b) maximal heat shock induction occurs at temperatures which are close to causing cell growth inhibition and, if prolonged substantially, cell death may occur, i.e. the cell counts of cultures will decline as a function of the number of heat shock cycles applied, resulting in diminishing yields

c) during most of the production time, the cells will be in a heat shock state in which many cellular activities are impeded (perhaps even glycosylation and transport of proteins) and would be expected to be kept at partially reduced levels

Recent advances in the understanding of the organization and natural expression control of HIV, have led to the description of a novel virus-encoded **trans**-activator protein defined as tat-III ( to distinguish from other comparable proteins such as tat-I and tat-II from human T-cell Leukemia viruses HTLV-I and HTLV-II) (Arya et al., (1985) Science 229, 69-73; Sodroski et al., (1985) Science 227, 171-173; Sodroski et al., (1985) Science 229, 74-77; Muesing et al., (1985) Nature 313, 450-458; Muesing et al., (1987) Cell 48, 691-701). The high level of viral expression in infected cells is attributed, at least in part, to tat-III. For instance, in a transient assay in human T cell lines infected with the HIV virus (previously HTLV-III) with the bacterial chloramphenicol acetyltransferase gene, the expression of this gene, when directed by the long terminal repeat sequences of this virus, is stimulated more than 300-fold as compared with the case of uninfected cells. It is assumed that this **trans**-activator protein interacts, either directly or indirectly, with DNA target sequences near the start of the transcription site of the viral promoter (Rosen at al., (1986) Nature 319, 555-559) which is part of long terminal repeats, i.e. the so-called LTRs. Tat-III also appears to act at the post-transcriptional level (Rosen et al., (1986) ibid). Recent work has suggested that the tat-III gene is required for the production of infectious HIV virions, although its precise role in viral replicative cycle remains unclear (Fischer et al., (1986) Nature 320, 367-371; Dayton et al., (1986) Cell 44, 941-947).

It has been also demonstrated that other human T cell leukemia viruses (HTLV) contain **trans**-acting transcriptional activators which act on their own LTR promoter sequences, see Sodroski et al., (1984) Science 225, 381-385; Fujisawa et al., (1985) PNAS 82, 2277-2281; Seiki et al., (1986) EMBO J. 5, 561-565 for HTLV-I and Greene et al., (1986) Science 232, 877-880 for HTLV-II. Similar observations were obtained on bovine leukemia virus (Rosen et al., (1985) Cell 41, 813-823) and on visna virus (Hess et al., (1985) Science 229, 482-485) LTR's. In addition, the same sequences appear to be responsible for the induction of certain cellular genes, such as Interleukin-2 and its receptor (Cross et al., (1987) Cell 49, 47-56; Maruyama et al., (1986) Cell 48, 343-350).

In addition to cells where the HIV is replicated, tat-III-activated LTR promoter are active in a variety of cells such as B and T lymphoid cell lines (Raji, Jurkat, H9 ...) and also in HeLa, CAS 3T3, CHO cells (Sodroski et al., 1985, Science 229, 74-77; Rosen et al., 1986, Nature 319, 555-559; Rosen et al., 1986, J. Virol 57, 379-384: Finberg et al., 1986, Cell 46, 808-817).

We have thus made use of the **trans**-activator phenomena, employing the HIV tat-III gene, to add a further optimization dimension to the previously described heat shock expression system (P. Bromley and R. Voellmy, (1983) Patent Application US Serial No. 628.588; P. Bromley et al., (1985) Patent Application PCT EP 86/00451; P. Bromley et al., (1986) European Patent Application No 86/810.455.5. The basic approach selected was to abandon the direct driving of the expression of genes of interest by heat shock promoters only; in contrast, as explained in claim 1 the genes of interest were placed under the control of HIV LTR promoter sequences, the latter becoming then stimulated by the **trans**-activator protein, the latter being produced in-situ. Thus, the LTR driven expression of these genes can then be regulated by the amount of tat-III protein produced in cells suitably chosen for specific protein production. Furthermore, the tat-III levels in these cells are regulated via a tat-III gene placed under the control of a human heat shock promoter that is co-introduced into cells together with the gene of interest. Naturally, the invention also includes the use. to drive the genes interest. of other promoters regulated by endogenous stimulating factors. e.g. tat-I and tat-II.

The advantages of this effective regulation cascade is as follows:-

1. After heat shock, tat-III mRNA and tat-III protein are made in quantities sufficient to over-stimulate the LTR promoter. Since the expression of genes of interest is expected to occur so long as when tat-III is present, even in very small quantity, expression of the genes of interest will continue for an extended period following heat shock, i.e. until the last molecules of tat-III have been exhausted in the cell metabolic machinery. The results given hereafter indicate that the tat-III activated LTR promoter is a powerful promoter system. It may well be superior to an activated heat shock promoter.

The combination of tight heat shock control of tat-III expression with the highly activatable LTR promoter appears to allow almost total inducible expression of a chosen gene at any time and, once production is initiated by a single heat shock of short duration, production will continue efficiently for an extended period of time.

The genes of interest which can be placed under the control of the HIV-LTR promoter are many and include viral antigens, blood factors, hormones, enzymatically active proteins, structural proteins, proteins for diagnostic tests etc., for example Human Hepatitis surface Antigen (HBsAg); tissue Plasminogen Activators (tPA); human Growth Hormone (hGH): Tumor Necrosis Factor (TNF); Interleukin and other products of clinical and pharmaceutical interest.

The construction which results from placing the gene of interest under the transcriptional control of the HIV-LTR promoter sequence (hybrid gene 1) and that of the second hybrid gene (2) where the **trans**-activator gene is driven by a heat shock sequence are preferably introduced into host cells in the form of plasmid vectors. These plasmids are derived from usual known plasmids with or without selective marker genes for easier clone selection. Non limitative examples are provided hereafter. Other types of carriers known in the art, e.g. of viral origin, can also be used. The hybrid genes (1) and (2) can be part of one or separate vectors whereby incorporation into host cells is carried out by transfection or co-transfection. The invention is not limited to driving only one gene of interest under the control of an activator stimulated regularly sequence. Two or more genes of interest can be involved as well under the control of one or several (one for each gene) regulatory sequences.

Host cells are selected for their ability to grow under usual conditions. to accept hybrid genes (1) and (2) and not to impede their expression under the required operational conditions. Practically most of the recipient cells known in the art are suitable, such as CHO (Chinese hamster ovary cells). COS, HeLa, 3T3 cells, etc.

Multiplication of these cells can be effected by usual means with usual culture media: otherwise. multiplication can be effected in warm-blooded animals in the form of tumors, as disclosed in European Patent Application No 86/810.455.5.

In the present invention, the following variants are possible: For instance. as we and others have demonstrateed, the human hsp70 promoter has an extremely low basal level of expression at control temperature, and is strictly inducible even when using a potentialy amplifiable system. This is therefore readily applicable to the invention. As such amplifiable system. one can use that based on bovine papilloma virus (BPV) vectors (European Patent Application number 86810455.5), or that using CHO cell lines containing a high number of the hsp70-c-myc hybrid gene obtained by co-amplification of the dihydrofolate reductase gene in the presence of increasing concentration of methotrexate (Wurm et al.. (1986) PNAS 83, 5414-5418). One can also propose that the tat-III LTR-hybrid system can be regulated after gene amplification; For instance, one may first introduce an LTR driven hybrid gene into suitable host cells. effect gene amplification, and introduce in the final cell-line the heat-shock driven tat gene vector. In another variant. even the transcription units would be integrated in the cellular DNA or maintained in an episomal state (with vectors carrying BPV sequences).

It seems evident that the hsp promoter-tat-III hybrid gene might utilize promoters from different molecular weight hsp's and from eukaryotic cell of different origins (human, Drosophila. yeast. plant. etc...) as well as engineered hsp promoter variants and or synthetic promoters.

The present invention also applies to cases when a cell line expresses naturally one of the two transcription units; it becomes then sufficient. to achieve the object defined in the claims, to transfect these cells with the second transcription unit in order to obtain the high level synthesis of the gene of industrial interest.

In addition to the marker sequences (for instance antibiotic resistance genes) on the vectors used to carry the modified genes of the invention, said vector can also carry oncogenic sequences for strain identification. for instance Harvey C-Ras or BPV.

## DETAILED DESCRIPTION OF THE INVENTION AND EXAMPLES

### 1) Plasmid constructions

1.1 pLRT-cat containing the chloramphenicol acetyltransferase gene (cat) under the control of the HIV-LTR.

In an initial experiment , a test gene construct, pLTR-cat, was prepared by linking an HIV-LTR region to a DNA fragment coding for chloramphenicol acetyltransferase. This gene product is a conveniently assayed one that allows a rapid control of gene expression. DNA from plasmid HXB2-gpt (Fisher et al., (1986), Nature 220, 367-371) which carries the genome of an HIV provirus was digested with HindIII, ends were filled in, and the DNA was further digested with BamHI and XhoI. A XhoI to HindIII (filled) fragment from the 3' LTR region of the viral genome (coordinates 8475 to 9197, see nucleotide sequence of Ratner et al., (1985), Nature 313, 277-284) was isolated electrophoretically and ligated to the large XbaI (filled) XhoI fragment of plasmid pSV2-catXX DNA (see below). The mixture was used to transform **E. coli**, and thereafter, bacterial colonies carrying recombinants were isolated. Plasmid DNA was prepared from a number of these isolates and was analyzed by restriction digestion. LTR-cat was among the correct recombinants. Plasmid pSV2-catXX is a derivative of pSV2-cat (Gorman et al., (1982) PNAS 79, 6777-6781). It contains an XbaI site at the position of the unique HindIII site of pSV2-cat, and an XhoI site at the NdeI site of pSV2-cat.

1.2 HS -tat-III containing the tat-III gene under the control of the human hsp70 promoter.

A construct capable of expressing tat-III under the control of a human hsp70 promoter, HS-tat, was also prepared. Tat-III coding sequences from pHXB3-tat (Feinberg et al., (1986) Call 46, 807-817) was digested with BssHII, ends were filled in, the DNA was further digested with XhoI (see sequence of tat-III in Arya et al., (1985) Science 229, 69-73) and a tat-III gene segment of about 700 nt in length was isolated. Plasmid D285D DNA containing a human hsp 70 b-galactosidase fusion gene (nucleotide -285 to nucleotide + 113 from p 17 (R. Voellmy et al., (1985) PNAS 79, 1776-1780; fragment XbaI-XhoI from D88, see Amin et al., (1987) Mol. Cell Biol. 7, 1055-1062) was digested with XbaI (cuts at nt 113 of the hsp70 RNA leader region), ends were filled in, the DNA was digested further with SalI (cuts at the end of the b-galactosidase gene coding region), and the larger of the two digest fragments, containing human hsp70 promoter and RNA leader sequences, as well as 3' trailer and vector sequences was isolated and ligated to the above tat-III segment. Correct recombinants were produced and identified as above.

1.3 p285-cat

pD285D was digested with XbaI-XhoI. The 398-bp resulting fragment containing the 285-bp of 5' non-transcript and 113-bp of the RNA leader sequences of the human hsp70 was inserted in XhoI and XbaI sites of pSV2 catXX to give p285 cat, which contains a hsp70-cat fusion gene.

1.4 Human Growth Hormone (hGH) gene under the contol of the HIV-LTR.

In another example, the human growth hormone (hGH) gene was placed under the control of the HIV--LTR sequence as follows:
Plasmid p17hGHneo (P. Bromley et al., (1985) PCT/EP86/00451) was digested with SalI and XbaI, sites located between the hsp70 sequences and the hGH sequences. In parallel pLTR-cat was digested with XhoI

and XbaI; the 0.7-kb fragment containing the LTR sequences was isolated and inserted into the above fragment such that the resulting plasmid. p17LTRhGHneo, carries the human hsp70 and the LTR promoters behind the hGH gene, and includes the neomycin transcription unit from pSV2 neo (Southern and Berg, (1982) J. Mol. Appl. Gen. 1, 327-341).

In order that the performance, regarding hGH expression, of the p17LTR hGHneo under stimulation by the **trans**-activator protein be compared to that of the gene under direct heat shock promoter control, we used plasmid p17hGH neo as a control.

This plasmid contains the hGH gene placed under the expression control of the human hsp70 gene promoter; this plasmid construction has been previously described (see the above Bromley reference).

2.) Gene expression experiments)

2.1 LTR-cat expression

LTR-cat DNA was transfected into COS 7 monkey cells, or human HeLa cells, either alone, or together with pHXB-tat-III (Feinberg et al.,), a plasmid containing a tat-III cDNA gene that is expressed under the control of an HIV promoter.

The LTR-cat gene was found to direct efficiently the synthesis of cat in the presence but not in the absence of tat-III. The presence of cat and its concentration were ascertained by the technique of Gorman et al.. (1982), PNAS 79, 6777-6781.

HeLa cells were transfected by the **DEAE dextran technique**, either with 285-cat plasmid construction, a human hsp70-cat fusion gene, or with a mixture of LTR-cat and HS-tat-III. One day after transfection, half of the cultures were subjected to a 3 hour heat treatment at 42 5C, the remaining cultures were kept at 37 C. All cultures were then incubated further at 37 C. One day or five days after the heat treatment, cells were harvested, extracts were prepared, and cat levels were determined (Gorman et al.,(1982), PNAS 79, 6777-6781).

High levels of cat activity were found in cells containing 285-cat one day after heat treatment (Table 1). Essentially no activity was present in non heat-treated, transfected cells. Five days after heat treatment, only very low levels (5 fold drop) of cat activity were found in 285-cat-containing cells. This indicates that cat production did not continue for very long after the heat treatment. Much of the cat enzyme that had been produced on the first day, must have decayed after five days.

On day one, cat levels in heat-shocked cells that had received both the LTR-cat and the HS-tat-III plasmids were very high, exceeding considerably the level reached in 285-cat-containing cells. Comparison cells which had not been heated contained none. More important. after five days the cells still contained 80% of the cat activity recorded after one day. Thus, for practical industrial purposes. the stimulated LTR promoter efficiency is stronger than the human heat shock promoter alone: the combination of the two constructs thus provides a powerful, while totally inducible, gene expression system. Synthesis in cells in which cat production is under the influence of the product from the heat-regulated tat-III **trans**-activator gene continues for days after only one initial inducing heat treatment.

TABLE 1.

| Cat Activities In Transfected HELA Cells. | | | |
|---|---|---|---|
| plasmids | Induction | Days After Shock | |
| | | 1 | 5 |
| 285-cat | None<br>HS | 525<br>7914 | 598<br>14857 |
| LTR-cat/HS-tat-III | None<br>HS | 632<br>231756 | 2443<br>186427 |
| Mock | Co·HS | 370 | 325 |

2.2 Expression of Human Growth Hormone (hGH).

In a manner analogous to that disclosed in the previous Example for the cat gene, the expression of plasmids containing the hGH gene was analyzed. In these expression experiments COS monkey cells were selected, due to our experience indicating that transient expression of hGH is significant in these cells. Two groups of experiments were carried out. In the first group (Table 2), the cultured cells ( $4.10^6$ per 100 mm diameter dish in 10 ml Dulbecco's medium supplemented with 10% foetal calf serum, FCS) were transfected using the DEAE dextran procedure. Two days later, the test cells were heated for 2 hours at 43°C. (Controls were kept at 37°C). Then the amount of hGH produced was ascertained over a 5 days period (after 1, 3 and 5 days). Measurements were performed using the ELISA kit supplied by Sensibead EIA, Terumo Medical Corp. The media were replaced after each sampling. One trial involved plasmid p522 dhfr a b-galactosidase containing plasmid disclosed in PCT/EP86/00451.

TABLE 2:

| Secretion of hGH by transfected COS cells. | | | | | |
|---|---|---|---|---|---|
| plasmids | amount of DNA/dish (mg) | Induction | days after heat shock | | |
| | | | 1 | 3 | 5 |
| | | | ng of hGH/ml in suspension media | | |
| p17hGHneo +<br>HS-tat-III | 20<br>4 | None<br>HS | 0<br><5 | <5<br><5 | <5<br><5 |
| p17LTRhGHneo | 20 | None<br>HS | 14<br>30 | 40<br>70 | 21<br>45 |
| p17LTRhGHneo<br>+ HS-tat-III | 20<br>4 | None<br>HS | 36<br>100 | 70<br>165 | 52<br>100 |
| p522dhfr | 20 | HS | <1 | <1 | <1 |

In another group of experiments (Table 3), about $10^6$ cells or less were incubated on 60mm dishes in 5ml Dulbecco's medium supplemented with 10% FCS. Transfection was effected using the DEAE dextran procedure and the rest of the experiments was carried out as indicated in the first group of experiments. The third day after transfection, the cells were split into $75cm^2$ flasks. When the trials consisted of 2 or more repeats, the aliquots were pooled before measuring the hGH.

TABLE 3

| plasmids | amount of DNA dish (μg) | dish number | Days after heat shock | | | |
|---|---|---|---|---|---|---|
| | | | induction | 1 | 3 | 5 |
| | | | ng hGH/ml suspension media | | | |
| p17hGHneo | 5 | 2 | None | 0 | 2 | 0 |
| | | 3 | HS | 2 | 1 | 0 |
| p17hGHneo HS-tat-III | 5 5 | 2 3 | None HS | 0 3 | 3 1 | 0 0 |
| p17LTRhGHneo | 5 | 2 | None | 7 | 14 | 8 |
| | | 3 | HS | 8 | 25 | 10 |
| p17LTRhGHneo HS-tat -III p17LTRhGHneo HS-tat-III 522dhfr | 5 1 5 5 5 | 2 3 2 3 2 | None HS None HS HS | 8 35 12 25 0 | 33 64 54 64 0 | 9 15 13 13 <2 |

The results from Tables 2 and 3 fully confirm the predictions. It can be seen that hGH synthesis and secretion into the growth media are virtually zero with p522dhfr after heat shock. In the absence of heat shock, the response of p17hGHneo is also negative although not negligible after heat shock. Cells transfected with p17LTRhGHneo produces hGH at a significant level without heat induction. and it is seen that this production continues the third and fifth days after transfection. A significant increase in the levels of expression is obtained when the cells are subjected to a single heat shock and this increase is apparent after each of the periods assayed. When the plasmids p17LTRhGHneo and HS-tat-III are cotransfected into COS cells at a weight ratio of 5:1. hGH expression levels at control temperatures are comparable to those obtained when the hGH-containing plasmid is employed alone. After a single heat shock of the cells transfected with both plasmids, a significant increase in hGH expression is observed. and this increase is sustained throughout the five days of the experiment.

We conclude from the above experiments that the initial results obtained using the cat gene are largely reproduced when this gene is replaced with a gene for a Eucaryotic, secretable protein, hGH. A significant difference between the results with the two genes results presumable from the use of HeLa cells for cat expression, and COS cells for the hGH expression. As stated above, COS cells were chosen for hGH expression, and this due to the fact that plasmid vectors carrying and origin of replication derived from SV40 virus are able to be highly amplified in these cells (Gluzman Y., 1982, Cold Spring Harbor Loboratory Press, NY). This choice of cell for transient expression is necessitated by the difference in sensitivity of the tests employed for assaying cat and hGH production. The rather lower difference in production levels of hGH at heat shock over control levels as compared to cat are probably a result partly of amplification of the hGH containing plasmid, and on the ratio of each plasmid that is effectively taken up by individual COS cells. We have indeed observed variations in the ratio of hGH production in COS cells when different ratios of each co-transfecting plasmid is employed.

Initial attempts to explore this point are confirmed by the results of Table 3. In this independent series of experiments. one observes that the results obtained for the expression of hGH in COS monkey cells are very similar to those seen in Table 2. Quantitative differences exist, due simply to the experimental conditions of repeating transient expression assays in COS cells. but from a qualitative point of view the effect of the heat shock expression of tat-III clearly increases the procution of hGH when this gene is placed under the control of an LTR. The relatively important expression of hGH simply under the control of an LTR (p17LTRhGHneo) may indicate the basal level of this promoter, at least under the conditions of plasmid amplification that occurs in COS cells.

The higher levels of hGH production when this plasmid is co-transfected together with two weight ratios of HS-tat-III (1:1, and 5:1), and in the absence of a heat shock is probably the result of a slight leakage of the heat-shock control in HS-tat-III. One again this result may be an exaggeration due to plasmild amplification, and it should be noted that apparently very low amounts of tat-III product seem to have a considerable effect on LTR activity. The considerably tighter control that was observed for the expression of cat in HeLa cells would appear to support this interpretation of the COS cell results. Nevertheless, there is clear demonstration in each of the sets of data in Tables 1, 2 and 3 that the expression system described in this invention is both considerably more powerful than the use of a heat shock promoter alone. In addition, the practical advantage of continued expression for some days after an initial heat shock once cells are returned to their normal growth temperature will be of great importance in the commercial scale up of the production of many Eucaryotic proteins.

## Claims

1. A method of producing proteins encoded by genes of interest placed under the expression control of a promoter regulated by an endogenous activation factor, comprising:

1) synthesizing a first hybrid gene incorporating said gene of interest under the transcriptional control of a promoter sequence capable of being stimulated by an activator protein;

2) synthesizing a second hybrid gene incorporating the gene for expressing said activator protein under the control of a enkaryotic inducible promoter;

3) selecting a suitable eukaryotic host cell line for expressing therein said first and second hybrid genes.

4) introducing said first and second hydrid genes into said host cells, subjecting them to selection and multiplication and applying stress to the multiplied cells, whereby the activator protein is produced by said second gene to stimulate the expression of said first gene, the amount of said activator produced consecutive to said stress being sufficient to promote the production of said protein of interest for a period considerably longer than the time after said second gene has reverted to normal.

2. The method of claim 1, in which the promoter in the first gene is a retroviral long terminal repeat (LTR) sequence and the activator protein is a trans-activator protein, both derived from HIV, and the inducible promoter is a enkaryotic heat shock promoter.

3. The method of claim 2, in which the hybrid genes are carried by DNA transfer vectors which are introduced into said host cells by transfection and/or infection.

4. The method of claim 3, in which the DNA vectors are plasmids, cosmids or virus DNA.

5. The method of claim 3, in which hybrid genes (1) and (2) are carried by two independent or only one transfer vector.

6. The method of claim 3, in which hybrid gene (1) belongs to a plasmid pLTR-cat prepared by linking an HIV-LTR region of about 0.7 Kb to a DNA fragment coding for chloramphenicol transferase, cat, and hybrid gene (2) belongs to a plasmid HS-tat-III prepared from a tat-III gene of a plasmid carrying the genome of an HIV provirus linked to a human hsp70 gene promoter and RNA leader sequence.

7. The method of claim 3, in which the plasmid carrying hybrid gene (1), p17LTRhGHneo, contains a HIV-LTR region of about 0.7 Kb associated with the human growth hormone (hGH) gene and hybrid gene (2) belongs to the HS-tat-III plasmid.

8. The method of claim 2, in which said host cells are multiplicated by cultivation in-vitro in culture media, or in-vivo in warm-blooded animal.

9. A DNA system for the expression of a protein of interest in suitably chosen host cells comprising (1) a first genetically engineered structure consisting of one or more genes coding for that protein under control of a long terminal repeat (LTR) promotor sequence from HIV, itself stimulated by a trans-activator protein, and (2) a second structure consisting of a gene for the in-situ expression of this trans-activator protein, under the driving action of a linked heat shock promotor of eukaryotic origin, both structure (1) and (2) being incorporated in said host cells.

10. The DNA system of claim 9, in which both (1) and (2) belong to a sole recombinant DNA construct carried on one sole DNA vector.

11. The DNA system of claim 9, in which structures (1) and (2) belong to two independent DNA constructs of two separate transfer vectors.

12. The DNA system of claim 10 or 11 in which the transfer vectors are plasmids, cosmids of viral sequences.

13. The DNA system of claim 12, in which the transfer vectors carry oncogenic or antibiotic resistance sequences for strain identification during cloning.

14. The DNA system of claim 9, comprising, in addition, a sequence for gene amplification, e.g. the dihydroxyfolate gene operating in the presence of methotrexate.

15. The DNA system of claims 10-14, in which the DNA vector carries, in addition, episomal replication sequences.

16. The DNA system of claim 9. in which said trans-activator gene is selected from tat-I, tat-II and tat-III.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | GENE, vol. 49, no. 1, January 1986, pages 1-8, Elsevier Science Publishers B.V., Amsterdam, NL; M. DREANO et al.: "High-level, heat-regulated synthesis of proteins in eukaryotic cells" * Whole article * | 1-16 | C 12 N 15/00 |
| Y | NATURE, vol. 319, no. 6054, February 1986, pages 555-559, London, GB; C.A. ROSEN et al.: "Post-transcriptional regulation accounts for the trans-activation of the human T-lymphotropic virus type III" * Whole article, especially the discussion * | 1-16 | |
| A | PROC. NATL. ACAD. SCI. USA, vol. 83, August 1986, pages 5414-5418; F.M. WURM et al.: "Inducible overproduction of the mouse c-myc protein in mammalian cells" * Page 5415, last paragraph; page 5416 * | 1-16 | TECHNICAL FIELDS SEARCHED (Int. Cl.4) C 12 N C 12 P |
| A | WO-A-8 505 636 (DANA-FABER CANCER INSTITUTE) * Pages 4,5 * | 1-16 | |

The present search report has been drawn up for all claims

| Place of search | Date of completion of the search | Examiner |
|---|---|---|
| THE HAGUE | 02-03-1988 | CUPIDO M. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 

& : member of the same patent family, corresponding
   document

EPO FORM 1503 03.82 (P0401)